# FASCICULE DE BREVET EUROPEEN

(11) **EP 1 773 765 B1**
(45) Date de publication et mention de la délivrance du brevet: **23.04.2008**
(21) Numéro de dépôt: 05788450.4
(22) Date de dépôt: 12.07.2005
(51) Int. Cl.: C07D 201/14

(54) **PROCEDE DE PURIFICATION PAR HYDROGENATION CATALYTIQUE DE LACTAME CONTENANT DES IMPURETES CHLOROLACTAMES**
VERFAHREN ZUR KATALYTISCHEN HYDRIERUNGSREINIGUNG VON CHOROLACTAMVERUNREINIGUNGEN ENTHALTENDEM LACTAM
METHOD FOR CATALYTIC HYDROGENATION PURIFICATION OF LACTAM CONTAINING CHOROLACTAM IMPURITIES

(30) Priorité: 16.07.2004 FR 0407918
(43) Date de publication de la demande: 18.04.2007
(73) Titulaire: ARKEMA FRANCE, 92700 Colombes (FR)
(72) Inventeur: HUB, Serge, F-69100 Villeurbanne (FR); LACROIX, Eric, F-69480 Amberieux d'Azergues (FR); BONNET, Philippe, F-69007 Lyon (FR); DEVIC, Michel, F-69110 Saint Foy Lès Lyon (FR)
(74) Mandataire: Mouttet, Marie-Paule
(86) Numéro de dépôt international: PCT/FR2005/001794
(87) Numéro de publication internationale: WO 2006/016060

(56) Documents cités:
- DATABASE CA [Online] CHEMICAL ABSTRACTS SERVICE, COLUMBUS, OHIO, US; NAKAZATO, KENSHO ET AL: "Purifying crude lactams" XP002319662 extrait de STN Database accession no. 1973:3748 cité dans la demande & JP 470 376 33B B4 (TORAY INDUSTRIES, INC.) 22 septembre 1972 (1972-09-22)
- DATABASE CA [Online] CHEMICAL ABSTRACTS SERVICE, COLUMBUS, OHIO, US; NICHIMURA, MICHIO ET AL: "Purification of lactam" XP002319663 extrait de STN Database accession no. 1971:509881 cité dans la demande & JP 460 237 43B B4 (TORAY INDUSTRIES, INC.) 7 juillet 1971 (1971-07-07)

## Description

### Domaine de l'invention.

La présente invention se rapporte à un procédé de purification de lactames cycliques contenant des impuretés chlorolactames par réaction d'hydrogénation catalytique.

### Art antérieur et problème technique.

La préparation de polyamides de haute qualité nécessite de disposer de lactames très purs. Des impuretés en faibles quantités, à peine décelables par l'analyse, provoquent une diminution de la qualité des polyamides.

Des polyamides préparés à partir de lactames impurs montrent ainsi une tendance au jaunissement et présentent un large spectre de poids moléculaires.

De plus, la nature des impuretés dépend du procédé de synthèse du lactame utilisé. Ainsi, le lactame préparé par photo-oximation contient des impuretés chlorées spécifiques de ce type de procédé (chlorolactames), dont la présence dégrade les propriétés des polymères fabriqués.

L'élimination de ces impuretés chlorées est donc indispensable. Une des techniques de purification bien connue de l'homme de l'art est de traiter le lactame avec de l'hydrogène sur des catalyseurs d'hydrogénation. Cette réaction élimine le chlore sous forme d'HCl et permet ainsi de transformer les chlorolactames en lactames.

Cependant, selon le brevet JP Sho 47-37632/72, les catalyseurs d'hydrogénation classiques comme le Nickel de Raney se désactivent assez rapidement. Selon les auteurs, l'origine de cette désactivation est directement liée à la faible solubilité de l'HCl co-produit dans les milieux réactionnels traditionnellement utilisés (lactame/ solvants organiques), bloquant les sites actifs du catalyseur.

L'utilisation d'un catalyseur à base de nickel modifié par du manganèse, en plus de l'ajout d'un hydroxyde alcalin (pour neutraliser l'HCl produit) permet d'améliorer la durée de vie du système catalytique, sans pour autant être totalement satisfaisante.

Le brevet JP Sho 47-37633/72 propose un catalyseur à base de Ni et de BaF₂ déposé sur SiO₂. La durée de vie de ce catalyseur est meilleure que celle du catalyseur cité en référence, mais elle présente néanmoins une désactivation mesurable.

Les procédés de purification par hydrogénation nécessitent des solvants inertes vis-à-vis de l'hydrogène, ce qui empêche entre autres l'utilisation de composés aromatiques, qui sont en général de bien meilleurs solvants du lactame. Le brevet JP Sho 46-23743/71 décrit pourtant un procédé d'hydrogénation avec des solvants aromatiques. Pour éviter l'hydrogénation du solvant, il préconise d'ajouter une amine soluble dans le milieu solvant aromatique. L'amine sert juste à inhiber l'hydrogénation du solvant et peut donc être utilisée en faibles quantités par rapport aux impuretés chlorées présentes. De plus, un hydroxyde alcalin est ajouté au milieu en quantité au moins stoechiométrique par rapport au chlore organique initial afin de neutraliser l'acidité produite par la réaction.

Il apparaît donc que la purification de lactame par hydrogénation nécessite l'ajout d'une solution d'hydroxyde alcalin et éventuellement d'une amine lorsque le solvant du lactame est un aromatique.

Cependant, la présence de ces ajouts n'empêche pas la désactivation des catalyseurs utilisés.

Sans vouloir être lié par une théorie, on peut penser que la désactivation des catalyseurs s'effectuerait vraisemblablement par cristallisation des sels chlorés formés sur le catalyseur.

Il existe donc un besoin d'améliorer ces procédés de purification des lactames par hydrogénation des chlorolactames et en particulier d'empêcher la désactivation des catalyseurs d'hydrogénation utilisés.

### Résumé de l'invention

L'invention se rapporte à un procédé de purification de lactames cycliques contenant 6 à 12 atomes de carbone et contenant des chlorolactames comme impuretés, par réaction d'hydrogénation en présence d'un catalyseur métallique, d'un solvant et d'un composé ayant un reste -NH- dans lequel, le reste -NH- est celui du lactame présent ou formé pendant la réaction d'hydrogénation et le solvant organique est un liquide ionique capable de solubiliser le chlorhydrate de lactame formé.

Selon un mode préféré de réalisation on utilise dans le procédé de purification un solvant organique qui est un liquide ionique choisi parmi le groupe constitué par les liquides ioniques comprenant un dérivé dialkylimidazolium de formule I ou II 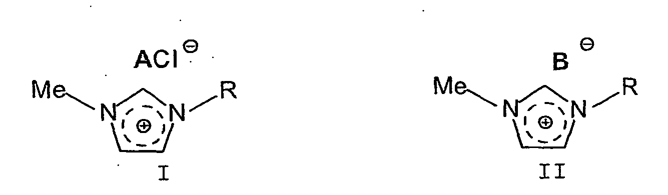 dans lesquelles
R est un groupe CH₃, - (CH₂)ₙ-CH₃, -CH₂-CₙF₂₊₁, phényl avec n étant un entier compris entre 1 et 10 , et
A représente AlCl₃, SbCl₃F₂, TaCl₅, TaF₅, NbCl₅, TiCl₄,
B représente SbF₆, BF₄, PF₆, CF₃SO₃.

Selon un mode de réalisation, le liquide ionique est formé par l'association entre le cation dialkyl imidazolium de formule I avec R tel que défini ci-dessus et un anion hexafluoroborate BF₆ ou bien un anion hexafluorophosphate PF₆.

Selon un mode de réalisation, le procédé est mis en oeuvre à une température comprise entre 100 et 250°C.

Selon un mode de réalisation, le lactame à purifier est le lauryl-lactame ou le caprolactame.

Selon un mode de réalisation, le lactame à purifier contient jusqu'à 5% en poids de chlorolactame.

Selon un mode de réalisation, l'étape d'hydrogénation s'effectue en faisant passer en continu un débit de lactame dans un solvant avec de l'hydrogène sur un catalyseur.

### Exposé détaillé de l'invention.

Les liquides ioniques utilisés dans l'invention sont décrits par exemple dans la demande de brevet n° WO 01/83353 (page 1 à page 4 et les exemples).

On préférera les liquides ioniques comprenant un dérivé dialkylimidazolium de formule I ou II 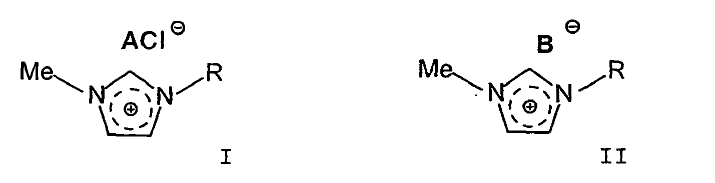 dans lesquelles
R est un groupe CH₃,- (CH₂)ₙ-CH₃, CH₂-CₙF₂ₙ₊₁, phenyl avec n étant un entier compris entre 1 et 10 , et
A représente AlCl₃, SbCl₃F₂, TaCl₅, TaF₅, NbCl₅, TiCl₄,
B représente SbF₆, BF₄, PF₆, CF₃SO₃.

On préfèrera encore les liquides ioniques formés par l'association entre le cation dialkyl imidazolium de formule I avec R tel que défini ci-dessus et un anion hexafluoroborate BF₆ ou bien un anion hexafluorophosphate PF₆.

Tout catalyseur d'hydrogénation à base de métaux déposés sur un support ou massique peut être utilisé dans le procédé, mais on préfère utiliser un catalyseur à base de Ni ou de Pd déposé sur alumine, silice, silice-alumine ou charbon.

Le procédé de purification selon l'invention peut être mis en oeuvre pour purifier les lactames cycliques contenant des chlorolactames comme impuretés, en particulier les lactames en C₆ (caprolactame), ou en C₁₂ (lauryl-lactame), monomères de base dans la synthèse des polyamides.

L'invention propose un procédé de purification de lactames cycliques contenant 6 à 12 atomes de carbone ayant des chlorolactames comme impuretés, par réaction d'hydrogénation en présence d'un catalyseur métallique, d'un solvant et d'un composé ayant un reste -NH- dans lequel, le reste -NH- est celui du lactame présent ou formé pendant la réaction d'hydrogénation et le solvant organique est choisi parmi les liquides ioniques tels que définis précédemment. Le liquide ionique est capable de solubiliser le chlorhydrate de lactame formé.

La présence du liquide ionique permet d'éviter la désactivation du catalyseur et d'améliorer les rendements de conversion des chlorolactames en lactames.

Selon un mode de réalisation préféré, le catalyseur métallique utilisé ici est le Pd/C ou le Ni/SiO₂.

Le liquide ionique préféré est le bmimPF₆ dans lequel le chlorhydrate de lactame, qui se forme durant la réaction d'hydrogénation entre l'HCl et le lactame est soluble à raison de 3% à 25°C et 35% à 100°C. De ce fait, le chlorhydrate de lactame solubilisé ne se dépose pas sur le catalyseur. La durée de vie du catalyseur, les rendements de conversion du chlorolactame en lactame sont ainsi améliorés.

Selon un autre mode de réalisation, le procédé de purification des lactames est un procédé en continu dans lequel on fait passer un débit du lactame à purifier de manière continue dans le solvant avec de l'hydrogène sur un catalyseur.

La vitesse volumique horaire peut être comprise entre 0,1 et 10 hr⁻¹, mais on opère préférentiellement entre 1 et 5 hr⁻¹.

### Exemples.

Les exemples suivants illustrent la présente invention sans toutefois en limiter la portée.

Le chlore organique initialement fixé à un atome de carbone du lauryllactame se retrouve après le traitement à l'hydrogène sous forme HCl relié au lactame lui-même. Un lavage à l'eau permet de récupérer cet HCl qui peut alors être dosé sous forme de chlorures.

On dose la quantité de chlore organique élémentaire initialement présent dans le lactame sous forme de chlorolauryllactame, la quantité de chlore organique élémentaire résiduel resté fixé sur le lactame et la quantité de chlorures récupérés dans les eaux de lavage, le tout exprimé en % en poids de chlore élémentaire.

On détermine la conversion des chlorolactames par le rapport;
% de chlore organique initial - % chlore organique résiduel
% de chlore organique initial.

On détermine le bilan chlore par le rapport
% chlore organique résiduel + % des chlorures récupérés
% chlore initial.

Un éventuel défaut de ce bilan chlore (écart par rapport à 100%) est synonyme d'accumulation de produit sur le catalyseur et donc de diminution de la durée de vie du catalyseur.

### Exemple 5 : avec liquide ionique et sans amine

Dans un autoclave en acier inoxydable on charge sous atmosphère d'argon pour être à l'abri de l'humidité:
50 g de bmimPF6, 2.64 g de catalyseur Pd / C et 5 g de lactame chloré (0.17 % de chlore total).

On pressurise l'autoclave avec 20 bar d'hydrogène et on chauffe le milieu réactionnel à 100°C pendant 6 heures sous agitation et sous 20 bar d'hydrogène.

Apres réaction on ajoute 50g de méthanol pour fluidifier le milieu et on filtre pour isoler le catalyseur.

Le méthanol est évaporé sous vide et lactame cristallisé est filtré et lavé avec un peu d'acétonitrile et avec de l'eau puis séché à l'étuve à 80°C.

Sa teneur en chlore total est de 0.005 % au lieu de 0.17 % avant traitement soit un taux de conversion du chlorolactame en lactame de 97%.

### Exemple 5c : comparatif

On procède exactement comme pour l'exemple 5 mais en remplaçant le liquide ionique par 50 g de cyclohexane.

La teneur en chlore total du lactame après traitement et lavage à l'eau est de 0.13 % au lieu de 0.17% avant traitement soit un taux de conversion du chlorolactame en lactame de 23.5%.

## Revendications

1. Procédé de purification de lactames cycliques contenant 6 à 12 atomes de carbone et contenant des chlorolactames comme impuretés, par réaction d'hydrogénation en présence d'un catalyseur métallique, d' un solvant et d' un composé ayant un reste-NH-dans lequel, le reste-NH-est celui du lactame présent ou formé pendant la réaction dhydrogénation et le solvant organique est un liquide ionique capable de solubiliser le chlorhydrate de lactame formé.

2. Procédé selon la revendication 1 dans lequel le solvant organique est un liquide ionique choisi parmi le groupe constitué par les liquides ioniques comprenant un dérivé dialkylimidazolium de formule 1 ou II 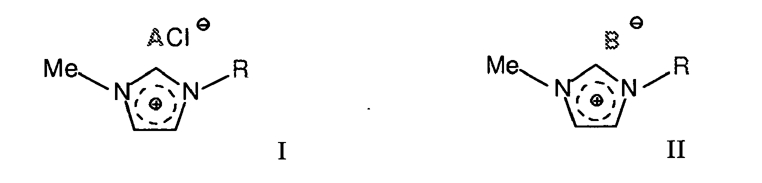 dans lesquelles R est un groupe CH₃,-(CH₂)n-CH₃,-CH₂-CnF₂ₙ₊₁, phényl avec n étant un entier compris entre 1 et 10, et A représente AlCl₃, SbCl₃F₂, Tacl₅, TaF₅, NbCl₅, TiCl₄, B représente SbF₆, BF₄, PF₆, CF₃SO₃.

3. Procédé selon la revendication 2 dans lequel le liquide ionique est formé par l' association entre le cation dialkyl imidazolium de formule I avec R tel que défini dans la revendication 2 et un anion hexafluoroborate BF₆ ou bien un anion hexafluorophosphate PF₆.

4. Procédé selon l'une des revendications 1 à 3 dans lequel la température est comprise entre 100 et 250°C.

5. Procédé selon l'une des revendications 1 à 4 dans lequel le lactame est le lauryl-lactame ou le caprolactame.

6. Procédé de purification selon l'une des revendications 1 à 5 dans lequel le lactame à purifier contient jusqu'à 5% en poids de chlorolactame.

7. Procédé de purification selon l'une des revendications 1 à 6 dans lequel l'étape d'hydrogénation s'effectue en faisant passer en continu un debit de lactame dans un solvant contenant éventuellement l'amine avec de l'hydrogène sur un catalyseur.

## Claims

1. Method for purifying cyclic lactams containing 6 to 12 carbon atoms and containing chlorolactams as impurities, by hydrogenation reaction in the presence of a metal catalyst, a solvent and a compound having a -NH- residue in which, the -NH- residue is that of the lactam present or formed during the hydrogenation reaction and the organic solvent is an ionic liquid capable of dissolving the lactam hydrochloride formed.

2. Method according to Claim 1, in which the organic solvent is an ionic liquid chosen from the group made up by ionic liquids comprising a dialkylimidazolium derivative of formula I or II 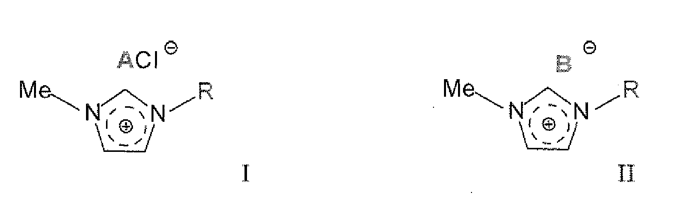 in which R is a CH₃, - (CH₂)ₙ-CH₃, -CH₂-CₙF₂ₙ₊₁ or phenyl group with n being an integer between 1 and 10, and A represents AlCl₃, SbCl₃F₂, TaCl₅, TaF₅, NbCl₅, TiCl₄, B represents SbF₆, BF₄, PF₆, CF₃SO₃.

3. Method according to Claim 2, in which the ionic liquid is formed by the association between the dialkylimidazolium cation of formula I with R as defined in claim 8 and a hexafluoroborate BF₆ anion or else a hexafluorophosphate PF₆ anion.

4. Method according to one of Claims 1 to 3, in which the temperature is between 100 and 250°C.

5. Method according to one of Claims 1 to 4, in which the lactam is lauryl lactam or caprolactam.

6. Purification method according to one of Claims 1 to 5, in which the lactam to be purified contains up to 5% by weight of chlorolactam.

7. Purification method according to one of Claims 1 to 6, in which the hydrogenation step is carried out by continuously passing a flow of lactam, in a solvent optionally containing the amine, with hydrogen over a catalyst.

## Patentansprüche

1. Verfahren zur Aufreinigung von cyclischen Lactamen mit 6 bis 12 Kohlenstoffatomen, die Chlorlactame als Verunreinigungen enthalten, durch Hydrierung in Gegenwart eines Metallkatalysators, eines Lösungsmittels und einer Verbindung mit einem -NH-Rest, wobei der -NH- Rest derjenige des vorliegenden oder während der Hydrierung gebildeten Lactams ist, und das organische Lösungsmittel eine ionische Flüssigkeit, die das gebildete Lactamhydrochlorid zu lösen vermag, ist.

2. Verfahren nach Anspruch 1, wobei das organische Lösungsmittel eine ionische Flüssigkeit ausgewählt aus der Gruppe der ionischen Flüssigkeiten umfassend ein Dialkylimidazoliumderivat der Formel I oder II 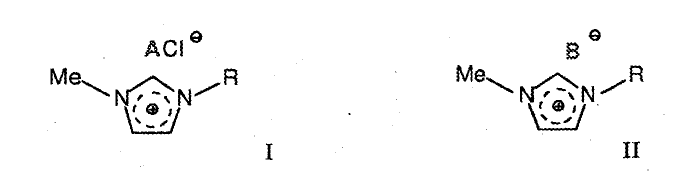 worin R eine Gruppe CH₃, -(CH₂)ₙ-CH₃, -CH₂-CnF₂ₙ₊₁ oder Phenyl, wobei n eine ganze Zahl zwischen 1 und 10 ist, bedeutet, A AlCl₃, SbCl₃F₂, Tacl₅, TaF₅, NbCl₅ oder TiCl₄ bedeutet und B SbF₆, BF₄, PF₆ oder CF₃SO₃ bedeutet, ist.

3. Verfahren nach Anspruch 2, wobei die ionische Flüssigkeit durch Vereinigung zwischen dem Dialkylimidazoliumkation der Formel I, wobei R wie in Anspruch 2 definiert ist, und einem Hexafluorboratanion BF₆ oder einem Hexafluorphosphatanion PF₆ gebildet wird.

4. Verfahren nach einem der Ansprüche 1 bis 3, wobei die Temperatur zwischen 100 und 250°C liegt.

5. Verfahren nach einem der Ansprüche 1 bis 4, wobei das Lactam Lauryllactam oder Caprolactam ist.

6. Aufreinigungsverfahren nach einem der Ansprüche 1 bis 5, wobei das aufzureinigende Lactam bis zu 5 Gew.-% Chlorlactam enthält.

7. Aufreinigungsverfahren nach einem der Ansprüche 1 bis 6, wobei der Hydrierungsschritt **dadurch** erfolgt, daß man eine Lactammenge kontinuierlich in ein Lösungsmittel einbringt, das gegebenenfalls das Amin mit dem Wasserstoff auf einem Katalysator enthält.
